(19) Europäisches Patentamt
European Patent Office
Office européen des brevets

(11) EP 4 435 042 A1

(12) EUROPEAN PATENT APPLICATION
published in accordance with Art. 153(4) EPC

(43) Date of publication:
**25.09.2024 Bulletin 2024/39**

(21) Application number: **23892061.5**

(22) Date of filing: **17.11.2023**

(51) International Patent Classification (IPC):
***C08J 11/24** (2006.01)* ***C08J 11/16** (2006.01)*
***C07D 317/38** (2006.01)* ***C07D 317/36** (2006.01)*
***C08J 11/18** (2006.01)* ***C08J 11/28** (2006.01)*

(52) Cooperative Patent Classification (CPC):
**C07D 317/36; C07D 317/38; C08J 11/16; C08J 11/18; C08J 11/24; C08J 11/28**

(86) International application number:
**PCT/KR2023/018534**

(87) International publication number:
**WO 2024/106998 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**18.11.2022 KR 20220155347**
**20.04.2023 KR 20230052249**
**16.11.2023 KR 20230159224**
**16.11.2023 KR 20230159225**

(71) Applicant: **LG Chem, Ltd.**
**Yeongdeungpo-gu**
**Seoul 07336 (KR)**

(72) Inventors:
- **LEE, Hyunyoung**
  **Daejeon 34122 (KR)**
- **PARK, Jun Beom**
  **Daejeon 34122 (KR)**
- **KIM, Hyun Cheol**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) METHOD FOR PREPARING RECYCLED CYCLIC CARBONATE COMPOSITION

(57) The present invention relates to a method for preparing a recycled cyclic carbonate composition, the method comprising the steps of: adding a polycarbonate-based resin to an organic solvent to prepare a mixed solution; adding an alkylene glycol and a catalyst to the mixed solution to depolymerize the polycarbonate-based resin; removing an aromatic diol compound, an organic solvent, and an alkylene glycol from the depolymerized solution; and recovering an alkylene carbonate from the depolymerized solution from which the aromatic diol compound, the organic solvent, and the alkylene glycol have been removed.



Processed by Luminess, 75001 PARIS (FR)

## Description

### [TECHNICAL FIELD]

CROSS-REFERENCE TO RELATED APPLICATIONS

This application claims the benefit of Korean Patent Application No. 10-2022-0155347 filed on November 18, 2022, Korean Patent Application No. 10-2023-0052249 filed on April 20, 2023, Korean Patent Application No. 10-2023-0159224 filed on November 16, 2023, and Korean Patent Application No. 10-2023-0159225 filed on November 16, 2023 in the Korean Intellectual Property Office, the disclosures of which are incorporated herein by reference in their entirety.

The present invention relates to a preparation method of a recycled cyclic carbonate composition recovered through recycling by chemical decomposition of a polycarbonate-based resin.

### [BACKGROUND]

Polycarbonate is a thermoplastic polymer and is a plastic having excellent characteristics such as excellent transparency, superior ductility, and relatively low production costs.

Although polycarbonate is widely used for various purposes, environmental and health concerns during waste treatment have been continuously raised.

Currently, a physical recycling method is carried out, but in this case, a problem accompanying the deterioration of the quality occurs, and thus, research on the chemical recycling of polycarbonate is underway.

Chemical decomposition of a polycarbonate refers to obtaining an aromatic diol compound (e.g., bisphenol A: BPA) and a carbonate compound (e.g., diethyl carbonate) which are monomers constituting polycarbonate through decomposition of a polycarbonate.

For such a chemical decomposition, thermal decomposition, hydrolysis, and alcohol decomposition are typically known. Among them, the most common method is alcohol decomposition, but in the case of methanol decomposition, there is a problem that methanol is used which is harmful to the human body, and in the case of ethanol, there is a problem that high temperature and high pressure conditions are required and the yield is not high.

Meanwhile, cyclic carbonates such as ethylene carbonate are useful as solvents for high molecular weight polymers and synthetic fuels for carbonate esters, and can also be applied as liquid electrolyte organic solvents for electric vehicle batteries. Conventionally, ethylene carbonate has been prepared through industrial synthesis using ethylene oxide and carbon dioxide, but there is a need to develop more environmentally friendly methods.

Therefore, as mentioned above, there is a growing need to develop a method for preparing industrially useful cyclic carbonates by chemical decomposition of a polycarbonate, taking advantage of the fact that carbonate compounds can be obtained through chemical decomposition of a polycarbonate.

### [DETAILED DESCRIPTION OF THE INVENTION]

### [Technical Problem]

It is an object of the present invention to provide a method for preparing a recycled cyclic carbonate composition in which a high-purity cyclic carbonate compound recovered through recycling by chemical decomposition of a polycarbonate-based resin can be secured with high yield.

### [Technical Solution]

In order to achieve the above object, provided herein is a method for preparing a recycled cyclic carbonate composition, the method comprising the steps of: adding a polycarbonate-based resin to an organic solvent to prepare a mixed solution; adding an alkylene glycol and a catalyst to the mixed solution to depolymerize the polycarbonate-based resin; removing an aromatic diol compound, an organic solvent, and an alkylene glycol from the depolymerized solution; and recovering an alkylene carbonate from the depolymerized solution from which the aromatic diol compound, the organic solvent, and the alkylene glycol have been removed.

Below, a method for preparing a recycled cyclic carbonate composition according to specific embodiments of the present invention will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context

clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

As used herein, the term "substituted or unsubstituted" means being unsubstituted or substituted with one or more substituent groups selected from the group consisting of deuterium; a halogen group; a cyano group; a nitro group; a hydroxy group; a carbonyl group; an ester group; an imide group; an amino group; a primary amino group; a carboxy group; a sulfonic acid group; a sulfonamide group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkoxysilylalkyl group; an arylphosphine group; or a heterocyclic group containing at least one of N, O and S atoms, or being unsubstituted or substituted with a substituent linking two or more substituent groups of the substituents illustrated above. For example, "a substituent linking two or more substituents" may be a biphenyl group. In other words, a biphenyl group may be an aryl group, or it may also be interpreted as a substituent linking two phenyl groups.

As used herein, the alkyl group is a monovalent functional group derived from an alkane, and may be a straight-chain or a branched-chain. The number of carbon atoms of the straight chain alkyl group is not particularly limited, but is preferably 1 to 20. Also, the number of carbon atoms of the branched chain alkyl group is 3 to 20. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, 2,6-dimethylheptane-4-yl and the like, but are not limited thereto. The alkyl group may be substituted or unsubstituted, and when substituted, examples of the substituent are the same as described above.

As used herein, the alkylene group is a bivalent functional group derived from alkane, and the description of the aryl group as defined above can be applied except that these are divalent functional groups. For example, the linear or branched alkylene group may include a methylene group, an ethylene group, a propylene group, an isobutylene group, a sec-butylene group, a tert-butylene group, a pentylene group, a hexylene group, and the like. The alkylene group can be substituted or unsubstituted, and when substituted, examples of the substituent are the same as described above.

As used herein, a fused ring means a cyclic structure wherein each of the adjacent rings shares only two atoms in a polycyclic system composed of two or more carbon rings or heterocycles.

As used herein, "one or more" means, for example, "1, 2, 3, 4 or 5, particularly 1, 2, 3 or 4, more particularly 1, 2 or 3, and even more particularly 1 or 2".

According to one embodiment of the present invention, there can be provided a method for preparing a recycled cyclic carbonate composition, the method comprising the steps of: adding a polycarbonate-based resin to an organic solvent to prepare a mixed solution; adding an alkylene glycol and a catalyst to the mixed solution to depolymerize the polycarbonate-based resin; removing an aromatic diol compound, an organic solvent, and an alkylene glycol from the depolymerized solution; and recovering an alkylene carbonate from the depolymerized solution from which the aromatic diol compound, the organic solvent, and the alkylene glycol have been removed.

The present inventors have found through experiments that although the recycled cyclic carbonate composition of the one embodiment was recovered through recycling by chemical decomposition of the polycarbonate-based resin, the composition has high purity at the level of a newly synthesized alkylene carbonate, thereby capable of realizing excellent physical properties and securing it in a high yield, and completed the present invention.

Specifically, according to the present invention, a polycarbonate is decomposed to an alkylene glycol under mild conditions, thereby being able to stably obtain an alkylene glycol, which is a monomer having high purity.

A method for preparing the recycled cyclic carbonate composition according to the one embodiment may comprise the steps of: adding a polycarbonate-based resin to an organic solvent to prepare a mixed solution, and adding an alkylene glycol and a catalyst to the mixed solution to depolymerize the polycarbonate-based resin.

The polycarbonate-based resin is meant to include both a homopolymer and a copolymer containing a polycarbonate repeating unit, and collectively refers to a reaction product obtained through a polymerization reaction or a copolymerization reaction of a monomer containing an alkylene carbonate and a carbonate precursor. When it contains one type of carbonate repeating unit obtained by using only one type of alkylene carbonate and one type of carbonate precursor, a homopolymer can be synthesized. In addition, when one type of alkylene carbonate and two or more types of carbonate precursors are used as the monomer, or two or more types of alkylene carbonates and one type of carbonate precursor are used, or one or more types of other diols are used in addition to the one type of alkylene carbonate and

the one type of carbonate precursor to contain two or more types of carbonates, a copolymer can be synthesized. The homopolymer or copolymer can include all of low-molecular compounds, oligomers, and polymers depending on the molecular weight range.

The polycarbonate-based resin can be applied regardless of various forms and types, such as a novel polycarbonate produced through synthesis, a recycled polycarbonate produced through a recycling process, or polycarbonate waste.

In the present invention, specific examples of the alkylene glycol are not particularly limited, and various known alkylene glycols can be used without limitation. Examples thereof include ethylene glycol or propylene glycol.

Meanwhile, the organic solvent may include one or more solvents selected from the group consisting of tetrahydrofuran, methylene chloride, chloroform, dimethyl carbonate, ethylmethyl carbonate, diethyl carbonate and dipropylcarbonate. That is, the organic solvent may include tetrahydrofuran, methylene chloride, chloroform, dimethyl carbonate, ethylmethyl carbonate, diethyl carbonate, dipropyl carbonate, or a mixture of two or more thereof.

Specifically, when methylene chloride is used as an organic solvent, there is an advantage that the dissolution properties in polycarbonate can be improved and the reactivity can be improved.

The weight ratio of the alkylene glycol, the organic solvent, and the polycarbonate-based resin is not particularly limited, however, for example, the weight ratio between the alkylene glycol: polycarbonate-based resin may be 10:1 to 1:10, or 1:1 to 1:10, or 1:1 to 1:5, or 1:1 to 1:2, or 1:1.1 to 1:2.

Further, the weight ratio between the organic solvent and the polycarbonate-based resin may be 10:1 to 1:10, or 10:1 to 1:1, or 10:1 to 2:1, or 10:1 to 5:1, or 10:1 to 6:1, or 10:1 to 7:1, or 10:1 to 8:1, or 9:1 to 8:1.

Further, the weight ratio between the organic solvent and the alkylene glycol may be 20:1 to 1:20, or 1:1 to 20:1, or 5:1 to 20:1, or 9:1 to 20:1, or 9:1 to 17:1.

Specifically, by mixing the alkylene glycol and the organic solvent within the above range, there is an advantage that the depolymerization reaction of the polymer can proceed at a desired level.

The catalyst may be added in an amount of 0.1 to 10 parts by weight, or 0.1 to 5 parts by weight, based on 100 parts by weight of the polycarbonate-based resin. Specifically, by containing the catalyst in the above content range, there is an advantage that economical catalytic reactions can proceed.

The catalyst may comprise one catalyst selected from the group consisting of an organic salt catalyst, an organic base catalyst, and an inorganic base catalyst. That is, the catalyst may include an organic salt compound, an organic base compound, an inorganic base compound, or a mixture of two or more thereof.

The organic salt catalyst may exist in the form of a salt containing an organic cation and an organic anion, and specifically, it may include an amidine-based salt compound. The amidine-based salt compound may include an amidine-based cation and an imidazole-based anion. The amidine-based cation may include all amidine cations and derivative cations thereof. The imidazole-based anion may include all imidazole anions and derivative anions thereof.

Specifically, the amidine-based salt compound may include an amidine-based cation having a fused ring. The fused ring may include a fused ring between a 7-membered ring and a 6-membered ring. The 7-membered ring means a case where the number of elements constituting the ring is 7, and the 6-membered ring means a case where the number of elements constituting the ring is 6.

The 6-membered ring may contain a C=N double bond of amidine. The 7-membered ring may contain a C-C single bond of amidine. Further, the C-N single bond of amidine may be included in the portion where the 7-membered ring and the 6-membered ring overlap.

More specifically, specific examples of the organic salt catalyst include a salt compound represented by the following Chemical Formula A:

[Chemical Formula A]

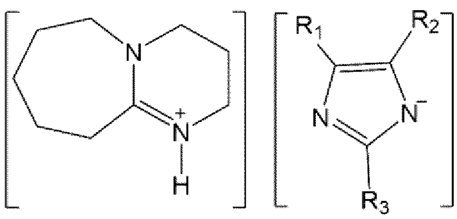

wherein, in Chemical Formula A, $R_1$, $R_2$, and $R_3$ are the same or different from each other, and may each independently be hydrogen or an alkyl.

Detailed examples of the organic salt catalyst represented by Chemical Formula A include Compound a where $R_1$=$R_2$=$R_3$=hydrogen(H) in Chemical Formula A, Compound b where $R_1$=$R_3$=hydrogen(H) and $R_2$=methyl($CH_3$) in Chemical Formula A, Compound c where $R_1$=$R_2$=hydrogen(H) and $R_3$=methyl($CH_3$) in Chemical Formula A, Compound d

where $R_1$=$R_3$=hydrogen(H) and $R_2$=isopropyl in Chemical Formula A, or Compound e wherein $R_1$=$R_2$=hydrogen(H) and $R_3$=isopropyl in Chemical Formula A, and the like, but are limited thereto.

On the other hand, the organic base catalyst means a nonionic organic compound having basicity, and specifically, it may include an amidine-based compound or a guanidine-based compound. The amidine-based compound may include all amidine compounds or derivative compounds thereof. The guanidine-based compound may include all guanidine compounds or derivatives thereof.

The amidine-based compound or the guanidine-based compound may have a fused ring. The fused ring may include a fused ring between a 6-membered ring and a 6-membered ring. Further, the fused ring may include a fused ring between a 7-membered ring and a 6-membered ring. Further, the fused ring may include a fused ring between a 5-membered ring and a 6-membered ring. The 7-membered ring means a case where the number of elements constituting the ring is 7, the 6-membered ring means a case where the number of elements constituting the ring is 6, and the 5-membered ring means a case where the number of elements constituting the ring is 5.

The 6-membered ring may contain a C=N double bond of amidine or guanidine. The 5-membered ring or 7-membered ring may contain a C-C single bond of amidine or a C-N single bond of guanidine. Further, the C-N single bond of amidine or guanidine may be included in the portion where two rings overlap.

Specifically, the amidine-based compound may include an amidine-based compound having a fused ring.

Detailed examples of the amidine-based compound include 1,8-diazabicyclo[5.4.0]undec-7-ene, 1,5-diazabicyclo[4.3.0]non-5-ene, or the like, but are not limited thereto.

Further, the guanidine-based compound may include a guanidine-based compound having a fused ring.

Detailed examples of the guanidine-based compounds include 1,5,7-triazabicyclo[4.4.0]dec-5-ene, 7-methyl-1,5,7-triazabicyclo[4.4.0]dec-5-ene, 1,2,3,5,6,7-hexahydroimidazo[1,2-a]pyrimidine, 1-methyl-1,2,3,5,6,7-hexahydroimidazo[1,2-a]pyrimidine, or the like, but are not limited thereto.

Meanwhile, the inorganic base catalyst means a nonionic inorganic compound having basicity, and specifically, it may be sodium hydroxide(NaOH) or potassium hydroxide(KOH), preferably sodium hydroxide(NaOH), without being limited thereto. The use of the inorganic base catalyst allows the decomposition reaction to proceed under mild conditions, which is economically advantageous.

Meanwhile, the step of adding an alkylene glycol and a catalyst to the mixed solution to depolymerize the polycarbonate-based resin may be stirring at 20°C to 100°C, or 50°C to 100°C, or 60°C to 100°C, or 70°C to 100°C for 1 hour to 24 hours, or 1 hour to 12 hours, or 1 hour to 8 hours.

Specifically, the conditions are mild process conditions as compared to the conventional pressurizing/high temperature process, and by performing stirring under the above conditions, the process can be performed in a mild process as compared to the pressurizing/high temperature process. In particular, when stirring at 70°C to 100°C for 1 to 12 hours, there is an advantage of obtaining the most efficient results in terms of reproducibility and acceptability.

Meanwhile, the method for preparing the recycled cyclic carbonate composition according to the one embodiment may comprise a step of removing an aromatic diol compound, an organic solvent, and an alkylene glycol from the depolymerized solution. Through the step of removing an aromatic diol compound, an organic solvent, and an alkylene glycol from the depolymerized solution, an alkylene carbonate in which the organic solvent, alkylene glycol, and aromatic diol compound are all separated and remain, can be obtained as the main product.

The step of removing an aromatic diol compound, an organic solvent, and an alkylene glycol from the depolymerized solution may comprise the steps of: removing the aromatic diol compound from the depolymerized solution; removing the organic solvent from the depolymerized solution; and removing the alkylene glycol from the depolymerized solution.

The order of the step of removing the aromatic diol compound from the depolymerized solution; the step of removing the organic solvent from the depolymerized solution; and the step of removing the alkylene glycol from the depolymerized solution, is not particularly limited, however, for example, the step of removing the aromatic diol compound from the depolymerized solution; the step of removing the organic solvent from the depolymerized solution; and the step of removing the alkylene glycol from the depolymerized solution can proceed in this order.

More specifically, the step of removing the aromatic diol compound from the depolymerized solution may comprise the steps of: crystallizing the aromatic diol compound formed by the depolymerization; and filtering the aromatic diol compound crystals.

The aromatic diol compound is characterized in that it is recovered from the polycarbonate-based resin. That is, this means that as a result of performing the recovery from the polycarbonate-based resin in order to obtain the recycled cyclic carbonate composition of the one embodiment, the aromatic diol compound is obtained together.

Specifically, "recovered from the polycarbonate-based resin" means being obtained through a depolymerization reaction of the polycarbonate-based resin. The depolymerization reaction can be carried out under acidic, neutral or basic conditions, and particularly, the depolymerization reaction can proceed under basic (alkaline) conditions. Particularly, the depolymerization reaction can be preferably carried out in the presence of a glycol-based compound, as will be described later.

Specific examples of the aromatic diol compound include bis(4-hydroxyphenyl)methane, bis(4-hydroxyphenyl)ether, bis(4-hydroxyphenyl)sulfone, bis(4-hydroxyphenyl)sulfoxide, bis(4-hydroxyphenyl)sulfide, bis(4-hydroxyphenyl)ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or a mixture of two or more thereof, and the like. Preferably, the aromatic diol compound may be 2,2-bis(4-hydroxyphenyl)propane (bisphenol A).

The step of crystallizing the aromatic diol compound formed by the depolymerization may proceed by adding the depolymerization solution to water. When the depolymerization solution is added to water, the aromatic diol compound contained in the depolymerized solution may be crystallized due to the difference in solubility. Crystallization conditions are not particularly limited, and various well-known crystallization conditions, methods, and equipment can be applied without limitation.

Among the aromatic diol compound, alkylene glycol, and alkylene carbonate contained in the depolymerized solution, the aromatic diol compound may form crystals, and the remaining materials may maintain a dissolved state. By filtering the aromatic diol compound crystallized at this time, the aromatic diol compound can be removed and an alkylene carbonate can be obtained.

In the step of filtering the aromatic diol compound crystals, the crystallized aromatic diol compound may be filtered under reduced pressure. Thereby, the aromatic diol compound contained in the depolymerized solution can be effectively removed. The filtration conditions are not particularly limited, and various well-known filtration conditions, methods, and equipment can be applied without limitation.

The step of removing the organic solvent from the depolymerized solution may include a step of distilling the organic solvent. The organic solvent may include one or more solvents selected from tetrahydrofuran, methylene chloride, chloroform, dimethyl carbonate, ethylmethyl carbonate, diethyl carbonate and dipropylcarbonate. That is, the organic solvent may include tetrahydrofuran, methylene chloride, chloroform, dimethyl carbonate, ethylmethyl carbonate, diethyl carbonate, dipropyl carbonate, or a mixture of two or more thereof. Specifically, when methylene chloride is used as an organic solvent, there is an advantage that the dissolution properties in polycarbonate can be improved and the reactivity can be improved.

The distillation temperature is adjusted to a temperature lower than the boiling point of the alkylene carbonate and higher than the boiling point of the organic solvent, so that the alkylene carbonate remains without being distilled, and the organic solvent can be removed by distillation. Other distillation conditions are not particularly limited, and various well-known distillation conditions, methods, and equipment can be applied without limitation.

Specifically, the step of distilling the organic solvent may proceed at a temperature of 40°C to 80°C, or 40°C to 70°C, or 40°C to 60°C, or 40°C to 50°C and under a pressure of 100 psi to 200 psi, or 120 psi to 180 psi, or 140 psi to 160 psi.

The step of removing the alkylene glycol from the depolymerized solution may comprise a step of adding the depolymerized solution to a solution separated into a water layer and an organic solvent layer, and removing an alkylene glycol dissolved in the water layer. The organic solvent may include one or more solvents selected from tetrahydrofuran, methylene chloride, chloroform, dimethyl carbonate, ethylmethyl carbonate, diethyl carbonate and dipropylcarbonate. That is, the organic solvent may include tetrahydrofuran, methylene chloride, chloroform, dimethyl carbonate, ethylmethyl carbonate, diethyl carbonate, dipropyl carbonate, or a mixture of two or more thereof. Specifically, when methylene chloride is used as an organic solvent, there is an advantage that the dissolution properties in polycarbonate can be improved and the reactivity can be improved.

Among the alkylene glycol and alkylene carbonate contained in the depolymerized solution, the alkylene glycol is relatively hydrophilic and can be dissolved in the water layer, and the alkylene carbonate can be dissolved in an organic solvent layer and separated into layers. At this time, the separated aqueous layer is removed to obtain an organic solvent layer, whereby the alkylene glycol can be removed to obtain an alkylene carbonate.

Meanwhile, the step of removing the aromatic diol compound, organic solvent, and alkylene glycol from the depolymerized solution may further comprise a step in which phenolic impurities derived from the aromatic diol compound formed by the depolymerization are removed with an adsorbent. The phenolic impurities may include one or more compounds selected from the group consisting of monohydroxyethyl-bisphenol A, bishydroxyethyl-bisphenol A, and tris(4-hydroxyphenyl)ethane.

Examples of the adsorbent that can be used include activated carbon, charcoal, celite, or a mixture thereof. The activated carbon is a black carbon material having micropores produced by subjecting a raw material to a carbonization process at about 500°C and an activated carbon process at about 900°C, and examples thereof are not particularly limited, however, for example, various activated carbons such as plant-based, coal-based, petroleum-based, waste-based activated carbon can be applied without limitation depending on the type of raw material.

By applying the adsorption purification process using an adsorbent, not only an alkylene carbonate, which is

the main synthesis target material in the present invention, be secured with high purity, but also the content of other impurities can be significantly reduced.

Adsorption purification conditions by the adsorbent are not particularly limited, and various well-known adsorption purification conditions can be used without limitation. However, as an example, the addition amount of the adsorbent may be 40% by weight to 60% by weight relative to the polycarbonate-based resin, and the adsorption time may be 0.1 hour to 5 hours. As for the adsorption method, stirring adsorption or a Lab adsorption tower can be used.

Meanwhile, the method for preparing the recycled cyclic carbonate composition of the one embodiment may comprise a step of recovering an alkylene carbonate from the depolymerized solution from which the aromatic diol compound, the organic solvent, and the alkylene glycol have been removed.

The step of recovering an alkylene carbonate from the depolymerized solution from which the aromatic diol compound, the organic solvent, and the alkylene glycol have been removed may comprise a step of distilling the depolymerized solution from which the aromatic diol compound, the organic solvent, and the alkylene glycol have been removed.

In the step of recovering an alkylene carbonate from the depolymerized solution from which the aromatic diol compound, the organic solvent, and the alkylene glycol have been removed, the organic solvent remaining in the depolymerized solution from which the aromatic diol compound, the organic solvent, and the alkylene glycol have been removed can be removed through distillation.

The distillation temperature is adjusted to a temperature lower than the boiling point of the alkylene carbonate and higher than the boiling point of the organic solvent, so that the alkylene carbonate remains without being distilled, and the organic solvent can be removed by distillation. Other distillation conditions are not particularly limited, and various well-known distillation conditions, methods, and equipment can be applied without limitation.

Specifically, the step of distilling the organic solvent may proceed at a temperature of 40°C to 80°C, or 40°C to 70°C, or 40°C to 60°C, or 40°C to 50°C and under a pressure of 100 psi to 200 psi, or 120 psi to 180 psi, or 140 psi to 160 psi.

**[Advantageous Effects]**

According to the present invention, there can be provided a method for preparing a recycled cyclic carbonate composition comprising an alkylene carbonate with a high purity and a high yield recovered through recycling by chemical decomposition of a polycarbonate-based resin.

**[DETAILED DESCRIPTION OF THE EMBODIMENTS]**

Hereinafter, the present invention will be explained in detail with reference to the following examples. However, these examples are for illustrative purposes only, and the scope of the present invention is not limited thereto.

<EXAMPLE>

Examples 1 to 5

266.67 g of Methylene chloride and 30 g of polycarbonate were added to a 250 ml three-neck flask, and then stirred.

Then, 21.83 g of ethylene glycol and 1.245 g of the catalyst listed in Table 1 below were added thereto, and subjected to a depolymerization reaction at 100°C for 3 hours.

When the depolymerization reaction was completed, the depolymerization reaction product was added to water to crystallize bisphenol A, which was then filtered under reduced pressure to remove bisphenol A. The resulting filtrate was then distilled at 40°C and 150 psi to remove methylene chloride, and then treated with charcoal to remove phenolic impurities.

The resulting filtrate was then separated into layers using water and methylene chloride, the water layer containing ethylene glycol was removed to obtain a methylene chloride layer containing ethylene carbonate. The resulting methylene chloride solution was distilled at 40°C and 150 psi to remove methylene chloride and obtain ethylene carbonate. Thereby, a recycled cyclic carbonate composition was prepared.

[Table 1]

| Category | Catalyst |
|---|---|
| Example 1 | Compound a |

(continued)

| Category | Catalyst |
|---|---|
| Example 2 | Compound b |
| Example 3 | Compound c |
| Example 4 | Compound d |
| Example 5 | Compound e |

- Compound a: in the following Chemical Formula A, $R_1=R_2=R_3$=hydrogen(H) 20ml
  0.6 g of imidazole and 1.5 g of 1,8-diazabicyclo(5,4,0)undec-7-ene were added a vial, and stirred at room temperature for 5 hours to obtain Compound a.
- Compound b: in the following Chemical Formula A, $R_1=R_3$=hydrogen(H), and $R_2$=methyl ($CH_3$)
  0.8 g of 2-methyl-1H-imidazole and 1.5 g of 1,8-diazabicyclo(5,4,0)undec-7-ene were added to a 20ml vial, and stirred at room temperature for 5 hours to obtain Compound b.
- Compound c: in the following Chemical Formula A, $R_1=R_2$=hydrogen(H), and $R_3$=methyl ($CH_3$)
  0.8 g of 4-methyl-1H-imidazole and 1.5 g of 1,8-diazabicyclo(5,4,0)undec-7-ene were added to a 20ml vial, and stirred at room temperature for 5 hours to obtain Compound c.
- Compound d: in the following Chemical Formula A, $R_1=R_3$=hydrogen(H), and $R_2$=isopropyl
  1.1 g of 2-isopropyl-1H-imidazole and 1.5 g of 1,8-diazabicyclo(5,4,0)undec-7-ene were added to a 20ml vial, and stirred at room temperature for 5 hours to obtain Compound d.
- Compound e: in the following Chemical Formula A, $R_1=R_2$=hydrogen(H), and $R_3$=isopropyl
  1.1 g of 4-isopropyl-1H-imidazole and 1.5 g of 1,8-diazabicyclo(5,4,0)undec-7-ene were added to a 20ml vial, and stirred at room temperature for 5 hours to obtain Compound e.

[Chemical Formula A]

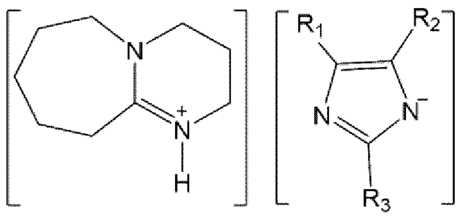

Examples 6 to 11

266.67 g of Methylene chloride and 30 g of polycarbonate were added to a 250 ml three-neck flask, and then stirred.

Then, 21.83 g of ethylene glycol and 0.783 g of the catalyst listed in Table 2 below were added thereto, and subjected to a depolymerization reaction at 100°C for 3 hours.

When the depolymerization reaction was completed, the depolymerization reaction product was added to water to crystallize bisphenol A, which was then filtered under reduced pressure to remove bisphenol A. The resulting filtrate was then distilled at 40°C and 150 psi to remove methylene chloride, and then treated with charcoal to remove phenolic impurities.

The resulting filtrate was then separated into layers using water and methylene chloride, the water layer containing ethylene glycol was removed to obtain a methylene chloride layer containing ethylene carbonate. The resulting methylene chloride solution was distilled at 40°C and 150 psi to remove methylene chloride and obtain ethylene carbonate. Thereby, a recycled cyclic carbonate composition was prepared.

[Table 2]

| Category | Catalyst |
|---|---|
| Example 6 | Compound 1 |
| Example 7 | Compound 2 |
| Example 8 | Compound 3 |

(continued)

| Category | Catalyst |
|---|---|
| Example 9 | Compound 4 |
| Example 10 | Compound 5 |
| Example 11 | Compound 6 |

- Compound 1: 1,5,7-triazabicyclo[4.4.0]dec-5-ene

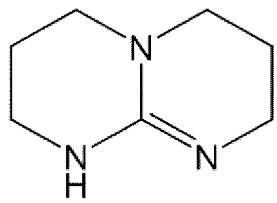

- Compound 2: 7-methyl-1,5,7-triazabicyclo[4.4.0]dec-5-ene

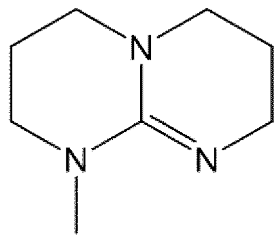

- Compound 3: 1,8-diazabicyclo[5.4.0]undec-7-ene

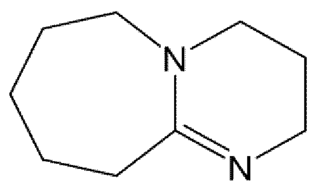

- Compound 4: 1,5-diazabicyclo[4.3.0]non-5-ene

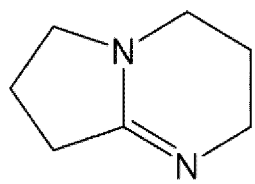

- Compound 5: 1,2,3,5,6,7-hexahydroimidazo[1,2-a]pyrimidine

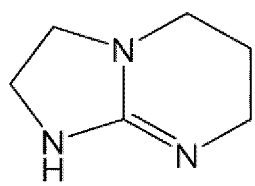

- Compound 6: 1-methyl-1,2,3,5,6,7-hexahydroimidazo[1,2-a]pyrimidine

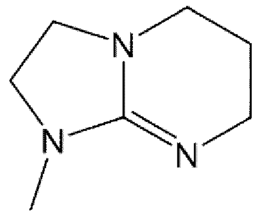

Example 12

266.67 g of Methylene chloride and 30 g of polycarbonate were added to a 250 ml three-neck flask, and then stirred.

Then, 21.83 g of ethylene glycol and 0.225 g of sodium hydroxide(NaOH) as a catalyst were added thereto, and subjected to a depolymerization reaction at 100°C for 3 hours.

When the depolymerization reaction was completed, the depolymerization reaction product was added to water to crystallize bisphenol A, which was then filtered under reduced pressure to remove bisphenol A. The resulting filtrate was then distilled at 40°C and 150 psi to remove methylene chloride, and then treated with charcoal to remove phenolic impurities.

The resulting filtrate was then separated into layers using water and methylene chloride, the water layer containing ethylene glycol was removed to obtain a methylene chloride layer containing ethylene carbonate. The resulting methylene chloride solution was distilled at 40°C and 150 psi to remove methylene chloride and obtain ethylene carbonate. Thereby, a recycled cyclic carbonate composition was prepared.

Example 13

A recycled cyclic carbonate composition was prepared in the same manner as in Example 12, except that the amount of ethylene glycol used was changed to 16.37g.

Example 14

Propylene carbonate was obtained in the same manner as in Example 12, except that 26.76g of propylene glycol was used instead of 21.83g of ethylene glycol, thereby preparing a recycled cyclic carbonate composition.

Example 15

Propylene carbonate was obtained in the same manner as in Example 12, except that 20.07g of propylene glycol was used instead of 21.83g of ethylene glycol, thereby preparing a recycled cyclic carbonate composition.

**<COMPARATIVE EXAMPLE>**

Comparative Example 1

60 ml of Methylene chloride, 30 ml of ethylene glycol, and 1.5 g of Compound a as a catalyst were added to a 250 ml three-neck flask, and stirred.

Then, 30 g of waste polycarbonate was added thereto, and the mixture was stirred at 40°C for 5 hours.

When the reaction was completed, ethylene glycol was distilled off, and then toluene was added thereto to separate bisphenol A and obtain ethylene carbonate. Thereby, a recycled cyclic carbonate composition was prepared.

Comparative Example 2

60 ml of Methylene chloride, 30 ml of ethylene glycol, and 1.5 g of Compound 1 as a catalyst were added to a 250 ml three-neck flask, and stirred.

Then, 30 g of waste polycarbonate was added thereto, and the mixture was stirred at 80°C for 36 hours.

When the reaction was completed, ethylene glycol was distilled off, and then toluene was added thereto to separate bisphenol A and obtain ethylene carbonate. Thereby, a recycled cyclic carbonate composition was prepared.

Comparative Example 3

60 ml of Methylene chloride, 30 ml of ethylene glycol, and 0.6 g of sodium hydroxide(NaOH) were added to a 250 ml three-neck flask, and stirred.
Then, 30 g of waste polycarbonate was added thereto, and the mixture was stirred at 40°C for 5 hours.
When the reaction was completed, ethylene glycol was distilled off, and then toluene was added thereto to separate bisphenol A and obtain ethylene carbonate. Thereby, a recycled cyclic carbonate composition was prepared.

Comparative Example 4

60 ml of Toluene, 30 ml of ethylene glycol, and 0.6 g of sodium hydroxide(NaOH) were added to a 250 ml three-neck flask, and stirred.
Then, 30 g of waste polycarbonate was added thereto, and the mixture was stirred at 60°C for 5 hours.
When the reaction was completed, ethylene glycol was distilled off, and then toluene was added thereto to separate bisphenol A and obtain ethylene carbonate. Thereby, a recycled cyclic carbonate composition was prepared.

Comparative Example 5

120 g of phenol and 40 g of polycarbonate were added to a 250 ml three-neck flask, and then stirred.
Then, 17.5 g of ethylene glycol and 1.245 g of Compound a as a catalyst were added thereto, and subjected to a depolymerization reaction at 80°C for 5 hours.
When the depolymerization reaction was completed, 100 g of toluene was added to the depolymerization reaction product, and the mixture was neutralized using hydrochloric acid, and distilled at 120°C and 150 psi to remove phenol, and then treated with charcoal to remove phenolic impurities. Then, the charcoal-treated material was added to water, crystallized, and filtered under reduced pressure to remove bisphenol A.
The resulting filtrate was then separated into layers using water and methylene chloride, the water layer containing ethylene glycol was removed to obtain a methylene chloride layer containing ethylene carbonate. The resulting methylene chloride solution was distilled at 40°C and 150 psi to remove methylene chloride and obtain ethylene carbonate. Thereby, a recycled cyclic carbonate composition was prepared.

Comparative Example 6

120 g of phenol and 40 g of polycarbonate were added to a 250 ml three-neck flask, and then stirred.
Then, 17.5 g of ethylene glycol and 0.783 g of Compound 1 were added thereto, and subjected to a depolymerization reaction at 80°C for 5 hours.
When the depolymerization reaction was completed, 100 g of toluene was added to the depolymerization reaction product, and the mixture was neutralized using hydrochloric acid, and distilled at 120°C and 150 psi to remove phenol, and then treated with charcoal to remove phenolic impurities. Then, the charcoal-treated material was added to water, crystallized, and filtered under reduced pressure to remove bisphenol A.
The resulting filtrate was then separated into layers using water and methylene chloride, the water layer containing ethylene glycol was removed to obtain a methylene chloride layer containing ethylene carbonate. The resulting methylene chloride solution was distilled at 40°C and 150 psi to remove methylene chloride and obtain ethylene carbonate. Thereby, a recycled cyclic carbonate composition was prepared.

Comparative Example 7

120 g of phenol and 40 g of polycarbonate were added to a 500 ml three-neck flask, and then stirred.
Then, 17.5 g of ethylene glycol and 1 g of sodium hydroxide (NaOH) as a catalyst were added thereto, and subjected to a depolymerization reaction at 80°C for 5 hours.
When the depolymerization reaction was completed, 100 g of toluene was added to the depolymerization reaction product, and the mixture was neutralized using hydrochloric acid, and distilled at 120°C and 150 psi to remove phenol, and then treated with charcoal to remove phenolic impurities. Then, the charcoal-treated material was added to water, crystallized, and filtered under reduced pressure to remove bisphenol A.
The resulting filtrate was then separated into layers using water and methylene chloride, the water layer containing ethylene glycol was removed to obtain a methylene chloride layer containing ethylene carbonate. The resulting methylene chloride solution was distilled at 40°C and 150 psi to remove methylene chloride and obtain ethylene carbonate. Thereby, a recycled cyclic carbonate composition was prepared.

**<Experimental Example>**

The physical properties of the recycled cyclic carbonate compositions obtained in Examples and Comparative Examples were measured by the following methods, and the results are shown in Table 1 below.

**1. Decomposition degree of polycarbonate** (%)

1 ml of the depolymerization reaction product obtained during the preparation process of the recycled cyclic carbonate composition was collected as a sample, high performance liquid chromatography(HPLC) analysis was performed under the following conditions, and the peak area ratio (unit: %) of bisphenol A relative to 100% of the total HPLC peak area was measured according to the following Equation 1:

Decomposition degree of polycarbonate (%) = (Bisphenol A area by HPLC / total peak area by HPLC) X 100 [Equation 1]

In Equation 1, the bisphenol A peak on HPLC corresponds to the peak with a retention time of 3.159 minutes, and the HPLC measurement conditions are as follows.

<HPLC conditions>

① Column: UG 120 (4.6mmI.DX50mm)
② Column Temp: 40°C
③ Injection volume: 10$\mu\ell$
④ Flow: THF:ACN:water=15:15:70, volume=1.23ml/min (total=10min)
⑤ Detector: 245nm

**2. Yield**

The weight of cyclic carbonate (ethylene carbonate, or propylene carbonate) produced when the polycarbonate used in the reaction was 100% decomposed was measured, and the weight of the obtained cyclic carbonate (ethylene carbonate, or propylene carbonate) was measured, so that the yield of the cyclic carbonate (ethylene carbonate, or propylene carbonate) was calculated as shown in the following Equation 2.

[Equation 2]

$$\text{Yield (\%)} = W_1 / W_O$$

wherein, in Equation 2, $W_0$ is the mass of the cyclic carbonate (ethylene carbonate, or propylene carbonate) obtained at the time of 100% decomposition, and Wi is the mass of the actually obtained cyclic carbonate (ethylene carbonate, or propylene carbonate).

[Table 3]

| Measurement results of Experimental Examples | | |
|---|---|---|
| Category | Decomposition degree of polycarbonate (%) | Yield (%) |
| Example 1 | 95.2 | 60.7 |
| Example 2 | 93.1 | 57.4 |
| Example 3 | 947 | 56.7 |
| Example 4 | 96.1 | 61.1 |
| Example 5 | 95.3 | 62.1 |
| Example 6 | 93.5 | 58.7 |

(continued)

| Measurement results of Experimental Examples | | |
|---|---|---|
| Category | Decomposition degree of polycarbonate (%) | Yield (%) |
| Example 7 | 967 | 59.9 |
| Example 8 | 93.4 | 55.7 |
| Example 9 | 94.5 | 60.8 |
| Example 10 | 947 | 61.2 |
| Example 11 | 95.6 | 62.1 |
| Example 12 | 94.57 | 6405 |
| Example 13 | 967 | 65.8 |
| Example 14 | 92.2 | 52.4 |
| Example 15 | 94.1 | 54.1 |
| Comparative Example 1 | 58.7 | 23.1 |
| Comparative Example 2 | 903 | 48.1 |
| Comparative Example 3 | 72.1 | 31.2 |
| Comparative Example 4 | 85.3 | 42.3 |
| Comparative Example 5 | 87.7 | 47.8 |
| Comparative Example 6 | 822 | 39.1 |
| Comparative Example 7 | 799 | 32.7 |

As shown in Table 3, the recycled cyclic carbonate compositions obtained in Examples 1 to 15 showed that the decomposition degree of polycarbonate was 92.2% to 96.7%, and the yield of cyclic carbonate (ethylene carbonate, or propylene carbonate) was 52.4% to 65.8%.

On the other hand, the recycled cyclic carbonate compositions obtained in Comparative Examples 1 to 7 showed that the decomposition degree of polycarbonate was 58.7% to 90.3% which was reduced as compared to those of Examples, and the yield of cyclic carbonate (ethylene carbonate, or propylene carbonate) was 23.1% to 48.1% which was reduced as compared to those of Examples.

## Claims

1. A method for preparing a recycled cyclic carbonate composition, the method comprising the steps of:

   adding a polycarbonate-based resin to an organic solvent to prepare a mixed solution;
   adding an alkylene glycol and a catalyst to the mixed solution to depolymerize the polycarbonate-based resin;
   removing an aromatic diol compound, an organic solvent, and an alkylene glycol from the depolymerized solution; and
   recovering an alkylene carbonate from the depolymerized solution from which the aromatic diol compound, the organic solvent, and the alkylene glycol have been removed.

2. The method for preparing the recycled cyclic carbonate composition according to claim 1, wherein:
   the organic solvent comprises one or more solvents selected from the group consisting of tetrahydrofuran, methylene chloride, chloroform, dimethyl carbonate, ethylmethyl carbonate, diethyl carbonate and dipropylcarbonate.

3. The method for preparing the recycled cyclic carbonate composition according to claim 1, wherein:
   a weight ratio between the organic solvent and the alkylene glycol is 20:1 to 1:20.

4. The method for preparing the recycled cyclic carbonate composition according to claim 1, wherein:
   the catalyst is added in an amount of 0.1 parts by weight to 10 parts by weight based on 100 parts by weight of the

polycarbonate-based resin.

5. The method for preparing the recycled cyclic carbonate composition according to claim 1, wherein: the catalyst comprises one catalyst selected from the group consisting of an organic salt catalyst, an organic base catalyst, and an inorganic base catalyst.

6. The method for preparing the recycled cyclic carbonate composition according to claim 5, wherein: the organic salt catalyst comprises an amidine-based salt compound.

7. The method for preparing the recycled cyclic carbonate composition according to claim 6, wherein:

the amidine-based salt compound comprises a salt compound represented by the following Chemical Formula A:

[Chemical Formula A]

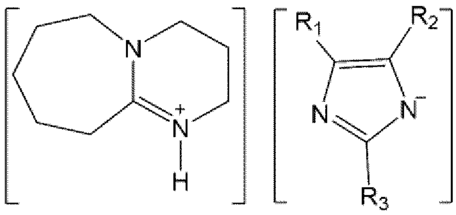

wherein, in Chemical Formula A, $R_1$, $R_2$, and $R_3$ are the same or different from each other, and each independently is hydrogen or an alkyl.

8. The method for preparing the recycled cyclic carbonate composition according to claim 5, wherein: the organic base catalyst comprises an amidine-based compound or a guanidine-based compound.

9. The method for preparing the recycled cyclic carbonate composition according to claim 8, wherein: the amidine-based compound comprises 1,8-diazabicyclo[5.4.0]undec-7-ene, or 1,5-diazabicyclo[4.3.0]non-5-ene.

10. The method for preparing the recycled cyclic carbonate composition according to claim 8, wherein: the guanidine-based compound comprises 1,5,7-triazabicyclo[4.4.0]dec-5-ene, 7-methyl-1,5,7-triazabicyclo[4.4.0]dec-5-ene, 1,2,3,5,6,7-hexahydroimidazo[1,2-a]pyrimidine, or 1-methyl-1,2,3,5,6,7-hexahydroimidazo[1,2-a]pyrimidine.

11. The method for preparing the recycled cyclic carbonate composition according to claim 5, wherein: the inorganic base catalyst comprises sodium hydroxide(NaOH) or potassium hydroxide(KOH).

12. The method for preparing the recycled cyclic carbonate composition according to claim 1, wherein: the adding an alkylene glycol and a catalyst to the mixed solution to depolymerize the polycarbonate-based resin is stirring at 20°C to 100°C for 1 hour to 30 hours.

13. The method for preparing the recycled cyclic carbonate composition according to claim 1, wherein: the removing an aromatic diol compound, an organic solvent, and an alkylene glycol from the depolymerized solution comprises the steps of:

removing the aromatic diol compound from the depolymerized solution;
removing the organic solvent from the depolymerized solution; and
removing the alkylene glycol from the depolymerized solution.

14. The method for preparing the recycled cyclic carbonate composition according to claim 13, wherein: the removing the aromatic diol compound from the depolymerized solution comprises the steps of:

crystallizing the aromatic diol compound formed by the depolymerization; and
filtering the aromatic diol compound crystals.

**15.** The method for preparing the recycled cyclic carbonate composition according to claim 13, wherein:

the removing the organic solvent from the depolymerized solution comprises,
distilling the organic solvent.

**16.** The method for preparing the recycled cyclic carbonate composition according to claim 15, wherein: the distilling the organic solvent is performed at a temperature of 40°C to 80°C and under a pressure of 100 psi to 200 psi.

**17.** The method for preparing the recycled cyclic carbonate composition according to claim 13, wherein:

the removing the alkylene glycol from the depolymerized solution comprises,
adding the depolymerized solution to a solution separated into a water layer and an organic solvent layer and removing alkylene glycol dissolved in the water layer.

**18.** The method for preparing the recycled cyclic carbonate composition according to claim 13, wherein:

the removing an aromatic diol compound, an organic solvent, and an alkylene glycol from the depolymerized solution further comprises
removing phenolic impurities derived from the aromatic diol compound formed by the depolymerization, with an adsorbent.

**19.** The method for preparing the recycled cyclic carbonate composition according to claim 1, wherein:

the recovering an alkylene carbonate from the depolymerized solution from which the aromatic diol compound, the organic solvent, and the alkylene glycol have been removed comprises,
distilling the depolymerized solution from which the aromatic diol compound, the organic solvent, and the alkylene glycol have been removed.

**20.** The method for preparing the recycled cyclic carbonate composition according to claim 19, wherein: the distilling the depolymerized solution from which the aromatic diol compound, the organic solvent, and the alkylene glycol have been removed is performed at a temperature of 40°C to 80°C and under a pressure of 100 psi to 200 psi.

## INTERNATIONAL SEARCH REPORT

International application No.
**PCT/KR2023/018534**

**A. CLASSIFICATION OF SUBJECT MATTER**

**C08J 11/24**(2006.01)i; **C08J 11/16**(2006.01)i; **C07D 317/38**(2006.01)i; **C07D 317/36**(2006.01)i; **C08J 11/18**(2006.01)i; **C08J 11/28**(2006.01)i

According to International Patent Classification (IPC) or to both national classification and IPC

**B. FIELDS SEARCHED**

Minimum documentation searched (classification system followed by classification symbols)

C08J 11/24(2006.01); B01J 31/02(2006.01); C07C 37/055(2006.01); C07C 37/72(2006.01); C07C 68/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 폴리카보네이트계 수지(polycarbonate-based resin), 해중합(depolymerization), 알킬렌글리콜(alkylene glycol), 방향족 디올 화합물(aromatic diol compound), 고리형 카보네이트(cyclic carbonate)

**C. DOCUMENTS CONSIDERED TO BE RELEVANT**

| Category* | Citation of document, with indication, where appropriate, of the relevant passages | Relevant to claim No. |
|---|---|---|
| X | QUARANTA, E. et al. Chemical recycling of poly(bisphenol A carbonate) by glycolysis under 1,8-diazabicyclo[5.4.0]undec-7-ene catalysis. ACS Omega. 2018, vol. 3, pp. 7261-7268.<br>See abstract; sections 2.3, 2.3.1 and 2.4; scheme 1; table 2; and page 7262. | 1-20 |
| A | KR 10-2022-0023451 A (LG CHEM, LTD.) 02 March 2022 (2022-03-02)<br>See entire document. | 1-20 |
| A | KR 10-2090680 B1 (INDUSTRIAL COOPERATION FOUNDATION JEONBUK NATIONAL UNIVERSITY) 18 March 2020 (2020-03-18)<br>See entire document. | 1-20 |
| A | 김동필 등. 폴리카보네이트의 글리콜첨가분해 / 메탄올첨가분해 복합 해중합. 청정기술. 2007, vol. 13, no. 4, pp. 251-256 (KIM, D. P. et al. Depolymerization of Polycarbonate Using Glycolysis/ Methanolysis Hybrid Process. Clean Technology.)<br>See entire document. | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 February 2024** | **20 February 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.
**PCT/KR2023/018534**

**C. DOCUMENTS CONSIDERED TO BE RELEVANT**

| Category* | Citation of document, with indication, where appropriate, of the relevant passages | Relevant to claim No. |
|---|---|---|
| A | JP 2009-108101 A (RES INST FOR PROD DEV) 21 May 2009 (2009-05-21)<br>See entire document. | 1-20 |

Form PCT/ISA/210 (second sheet) (July 2022)

**INTERNATIONAL SEARCH REPORT**
**Information on patent family members**

International application No.
**PCT/KR2023/018534**

| Patent document cited in search report | Publication date (day/month/year) | Patent family member(s) | Publication date (day/month/year) |
|---|---|---|---|
| KR 10-2022-0023451 A | 02 March 2022 | None | |
| KR 10-2090680 B1 | 18 March 2020 | KR 10-2020-0014004 A | 10 February 2020 |
| JP 2009-108101 A | 21 May 2009 | JP 2001-192497 A | 17 July 2001 |
| | | JP 5158973 B2 | 06 March 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020220155347
- KR 1020230052249
- KR 1020230159224
- KR 1020230159225